# FASCICULE DE BREVET EUROPEEN

(11) **EP 0 707 455 B1**
(45) Date de publication et mention de la délivrance du brevet: **14.05.1997**
(21) Numéro de dépôt: 94922891.0
(22) Date de dépôt: 08.07.1994
(51) Int. Cl.: A61B 17/32, A61B 17/36

(54) **BISTOURI A ULTRASONS**
ULTRASCHALLSKALPELL
ULTRASONIC SURGICAL KNIFE

(30) Priorité: 08.07.1993 FR 9308419
(43) Date de publication de la demande: 24.04.1996
(73) Titulaire: SATELEC S.A., F-33700 Merignac (FR)
(72) Inventeur: DIERAS, Francis, F-33000 Bordeaux (FR); BILLARD, Jean-Luc, F-33370 Tresses (FR)
(74) Mandataire: Bruder, Michel
(86) Numéro de dépôt international: FR9400853
(87) Numéro de publication internationale: WO9501754

(56) Documents cités:
- WO-A-90/04362
- WO-A-91/01692
- DE-A- 3 209 444
- DE-A- 3 707 921
- DE-U- 8 712 715
- US-A- 3 982 541
- US-A- 4 729 373

## Description

La présente invention concerne un bistouri, et plus particulièrement un bistouri laser à ultrasons destiné à réaliser la coupe, la dissection, la vaporisation et l'hémostase de tissus biologiques.

On sait que les bistouris à ultrasons sont utilisés en chirurgie pour assurer la fragmentation des tissus. Cette fragmentation peut être réalisée de façon sélective, en raison du phénomène de cavitation qui se manifeste sur les parties les plus hydratées des tissus, phénomène de cavitation que l'on peut moduler, au moyen d'une irrigation contrôlée et/ou d'un réglage de l'amplitude des vibrations.

On connaît, notamment par le brevet français n°85.11106 au nom de la demanderesse, un bistouri à ultrasons comportant des moyens d'irrigation des tissus à fragmenter et des moyens d'aspiration permettant de récupérer à la fois les tissus fragmentés et le liquide d'irrigation.

On sait également que si les bistouris à ultrasons sont appropriés pour réaliser une action sélective sur les tissus, leur pouvoir hémostatique est limité aux très petits vaisseaux, si bien que le chirurgien doit disposer de moyens propres à effectuer la cautérisation des tissus une fois la coupe de ceux-ci réalisée. Une telle opération de coupe nécessite donc l'utilisation d'un second instrument ce qui, dans le cas d'opérations complexes, notamment dans le cas de chirurgie endoscopique, présente de notables inconvénients, notamment en raison de la difficulté qui existe pour repositionner de façon précise et rigoureuse l'instrument de cautérisation, de façon que son outil se trouve dans la position initiale où se trouvait l'outil de coupe.

On a proposé de réaliser la cautérisation des tissus en utilisant la sonotrode de l'appareil à ultrasons en tant qu'électrode haute fréquence. Un inconvénient de ce type de technique est que la sonotrode, si elle est d'une forme adaptée à la propagation des ultrasons et aux phénomènes de fragmentation des tissus, possède un profil qui ne correspond pas à celui que l'on attend d'un appareil de coupe ou d'hémostase.

On a également utilisé, en tant que bistouri, des lasers qui réalisent une coupe nette des tissus ainsi que l'hémostase de ces derniers, mais qui présentent l'inconvénient, lorsqu'ils coupent, de ne pas dégager le champ opératoire. Par ailleurs, ces instruments présentent l'inconvénient de produire des fumées qui obscurcissent le champ opératoire et limitent de ce fait la précision du travail du chirurgien.

On connaît, par le brevet DE-A-2 707 921 un bistouri à ultrasons équipé d'un laser dont le rayonnement est guidé par une fibre optique jusqu'à l'extrémité antérieure du bistouri.

La présente invention a pour but de proposer un bistouri à ultrasons, équipé de moyens de coupe et de cautérisation à laser, qui permet d'éliminer les débris de carbonisation provenant de la découpe effectuée par celui-ci ainsi que les fumées produites au cours de cette découpe.

La présente invention a ainsi pour objet un bistouri comportant des moyens générateurs d'ultrasons aptes à soumettre une sonotrode, disposée à son extrémité antérieure, à un mouvement vibratoire ultrasonore, le bistouri étant percé d'au moins un canal axial d'aspiration reliant sa partie antérieure à sa partie postérieure, et comportant des moyens aptes à guider un rayonnement laser jusqu'à l'extrémité antérieure de la sonotrode, caractérisé en ce qu'il comporte des moyens pour diriger ledit rayonnement laser au travers du canal d'aspiration et pour le focaliser, en un point de focalisation situé en avant de l'extrémité antérieure de la sonotrode.

Dans un mode de mise en oeuvre intéressant de l'invention le bistouri comporte un canal d'aspiration débouchant dans le canal central et des moyens d'obturation de ce dernier disposés en amont du canal d'aspiration.

Dans un autre mode de mise en oeuvre intéressant de l'invention, le bistouri comporte des moyens de distribution d'un flux gazeux dans le second canal axial d'aspiration, qui débouchent dans celui-ci en amont du conduit d'aspiration, et qui créent, un courant gazeux antagoniste au courant d'aspiration.

On décrira ci-après à titre d'exemple non limitatif, une forme d'exécution de la présente invention, en référence au dessin annexé sur lequel :
La figure 1 est une vue en coupe longitudinale d'un premier mode de mise en oeuvre du bistouri sur l'invention.
La figure 2 est une vue en coupe longitudinale d'un second mode de mise en oeuvre du bistouri suivant l'invention en position de démontage de la pièce à main.

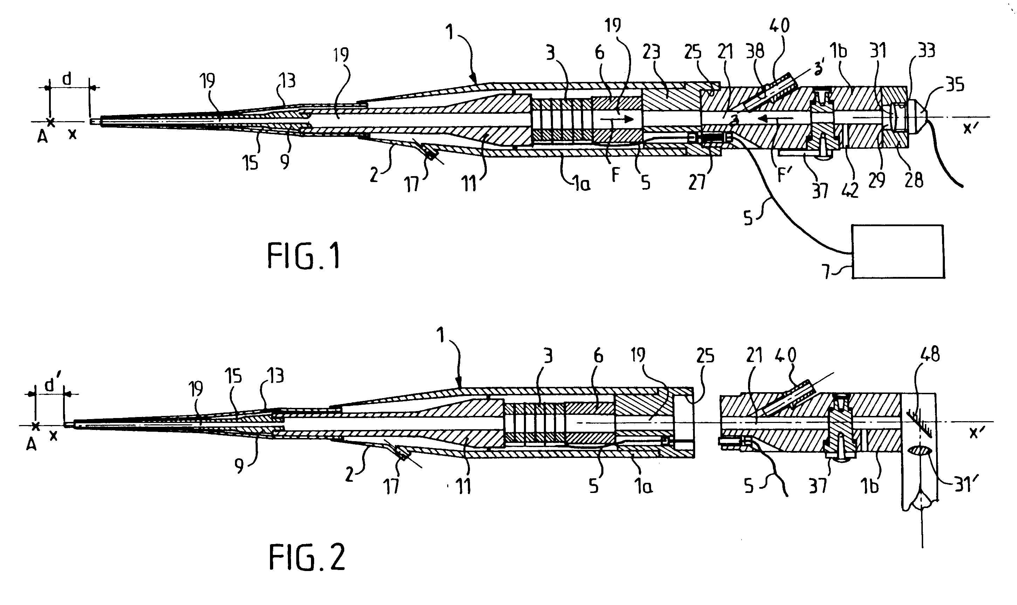

Sur les figures 1 et 2 le bistouri comprend essentiellement un corps 1 constitué d'une partie antérieure 1a, ou pièce à main, et d'une partie postérieure 1b ou connecteur. La pièce à main 1a se termine, sur sa partie antérieure, par une tête tronconique 2 qui renferme un transducteur 3 constituée d'un empilement d'éléments piézo-électriques qui sont alimentés en courant électrique par des fils d'alimentation 5 reliés à un générateur de courant haute fréquence 7. De façon connue, le transducteur 3 est en contact d'un côté avec une sonotrode 9, par l'intermédiaire d'une pièce d'amplification 11, et de l'autre côté avec une pièce d'appui ou "enclume" 6. La sonotrode 9 est entourée d'une enveloppe 13 qui définit entre elle-même et la sonotrode 9 un passage annulaire 15, alimenté en liquide d'irrigation par un conduit 17 relié à un réservoir d'irrigation, non représenté sur le dessin.

L'extrémité postérieure de la pièce à main 1a est fermée par un bouchon 23 solidaire de celle-ci, qui s'ouvre sur l'arrière par une cavité cylindrique 25 dans laquelle vient s'ajuster la partie postérieure du corps 1, ou connecteur 1b, avec interposition de moyens d'étanchéité, non représentés sur le dessin. La pièce à main 1a est traversée sur toute sa longueur par un canal axial 19 qui débouche d'un côté au niveau de la partie antérieure de la sonotrode 9 et, de l'autre côté, dans le fond de la cavité 25 du bouchon 23.

Des moyens de connexion électrique mâle/femelle 27 permettent de raccorder la pièce à main 1a et le connecteur 1b au générateur 7, par l'intermédiaire des fils d'alimentation 5. Le connecteur 1b se termine à sa partie postérieure par une bague 28 percée d'une cavité axiale 29 dans laquelle est encastrée une lentille de focalisation 31. La cavité axiale 29 comporte un filetage interne 33 dans lequel vient se fixer un élément guide d'onde 35 d'un laser, non représenté sur le dessin. Le connecteur 1b est traversé par un canal axial 21 qui se trouve dans le prolongement du canal axial 19 et qui est rigoureusement aligné avec celui-ci lorsque le connecteur 1b est fixé sur la pièce à main 1a. Dans ces conditions le bistouri est donc traversé de part en part par les deux canaux 19 et 21.

A faible distance en aval de la lentille de focalisation 31, le second canal axial 21 est pourvu d'un robinet obturateur 37 apte à occuper au moins deux positions, à savoir une position d'ouverture (figure 1) dans laquelle les canaux 19 et 21 sont dégagés de façon que le rayonnement émis par 1' élément guide d'onde 35 du laser puisse traverser les canaux 19 et 21, pour être focalisé en un point A de l'axe longitudinal xx' de l'appareil, situé à une distance d en avant de la sonotrode 9, et une position d'obturation (figure 2) dans laquelle le rayonnement émis par le laser n'est pas admis dans le canal 21.

En aval du robinet obturateur 37, le connecteur 1b est percé d'un orifice fileté 38 d'axe zz' incliné sur l'axe xx' et qui débouche dans le canal 21, dans lequel est vissé un embout 40 relié à des moyens d'aspiration, non représentés sur le dessin.

Entre la lentille de focalisation 31 et le robinet obturateur 37 on a prévu un conduit 42, de petit diamètre, par lequel le canal 21 communique avec l'extérieur.

Dans ces conditions, lorsque l'on souhaite utiliser l'appareil en tant que bistouri ultrasonore, on ferme le robinet obturateur 37, de façon à isoler du canal 21 la lentille de focalisation 31, ce qui la protège des projections diverses, puis on alimente en liquide d'irrigation le conduit 17 et l'on excite le transducteur 3 en assurant son alimentation en énergie électrique au moyen du générateur haute fréquence 7. On active également les moyens d'aspiration, raccordés à l'embout 40, de façon à pouvoir évacuer les résidus provenant de l'action du bistouri.

Lorsque le travail est effectué, et que l'on souhaite cautériser des tissus, on ouvre le robinet obturateur 37, de façon à dégager les canaux axiaux 19 et 21 et permettre le passage du rayonnement laser focalisé par la lentille 31.

Au cours du fonctionnement du laser, les fumées produites par l'action de celui-ci sur les tissus sont aspirées par les canaux 19 et 21 pour être évacuées par l'orifice 38 et l'embout 40. L'aspiration créée à partir de ce dernier induit une aspiration, par effet Venturi, qui crée un flux d'air de direction F', qui s'oppose au flux d'aspiration F, si bien que le flux F' formant un flux antagoniste empêche les fumées et autres résidus produits par l'action du faisceau laser de venir polluer la lentille de focalisation 31.

Bien entendu, le bistouri suivant l'invention peut être utilisé, non seulement pour désagréger des tissus par l'action des ultrasons et pour les cautériser au laser, mais également pour effectuer une découpe directe des tissus à l'aide du faisceau laser.

La présente invention est particulièrement intéressante en ce qu'elle permet au praticien, notamment dans le cas d'interventions sous contrôle endoscopique, après avoir positionné le bistouri une première fois et avoir effectué une opération sous ultrasons, de n'avoir pas à le repositionner une seconde fois pour réaliser l'intervention avec le laser, puisque le passage de l'un à l'autre se fait par un simple basculement d'éléments de commande.

Dans le mode de mise en oeuvre représenté sur les figures, les moyens de focalisation sont constitués d'une lentille 31, mais l'on pourrait bien entendu faire appel à tout autre moyen de focalisation différent.

On peut également, comme représenté sur la figure 2, utiliser un laser produisant un faisceau qui ne soit pas dans l'axe xx' des canaux axiaux 19 et 21, que l'on focalise au moyen d'une lentille 31'. Le faisceau est ensuite dirigé suivant l'axe xx' du bistouri au moyen d'un miroir 48, et la focalisation est réalisée en un point A de l'axe xx', à une distance d' donnée souhaitée située en avant de l'extrémité de la sonotrode 9.

## Revendications

1. Bistouri comportant des moyens générateurs d'ultrasons (3) aptes à soumettre une sonotrode (9), disposée à son extrémité antérieure, à un mouvement vibratoire ultrasonore, le bistouri étant percé d'au moins un canal axial d'aspiration (19,21) reliant sa partie antérieure à sa partie postérieure, et comportant des moyens aptes à guider un rayonnement laser jusqu'à l'extrémité antérieure de la sonotrode, caractérisé en ce qu'il comporte des moyens pour diriger ledit rayonnement laser au travers du canal d'aspiration (19,21) et pour le focaliser, en un point de focalisation (A) situé en avant de l'extrémité antérieure de la sonotrode (9).

2. Bistouri suivant la revendication 1 caractérisé en ce qu'il comporte un conduit d'aspiration secondaire (40) débouchant dans le canal axial d'aspiration (21), à proximité desdits moyens de focalisation (31) aptes à diriger le rayonnement laser en un point de focalisation (A), créant un courant d'aspiration (F), et des moyens aptes à commander l'obturation du canal axial d'aspiration, disposés en amont dudit conduit d'aspiration (40) et en aval des susdits moyens de focalisation (31)

3. Bistouri suivant l'une des revendications précédentes caractérisé en ce qu'il comporte des moyens de distribution d'un flux gazeux dans le canal axial d'aspiration (21), qui débouchent dans celui-ci, en amont du conduit d'aspiration (40), et qui créent, un flux gazeux (F') antagoniste au courant d'aspiration (F).

4. Bistouri suivant la revendication 3 caractérisé en ce que les moyens de distribution du flux antagoniste (F') au flux d'aspiration (F), sont constitués d'un conduit (42) reliant le canal axial (21) à l'extérieur, de façon à admettre ledit flux antagoniste par effet Venturi.

5. Bistouri suivant l'une des revendications précédentes caractérisé en ce que le bistouri est essentiellement constitué de deux éléments séparés connectables, à savoir un élément antérieur (1a), ou pièce à main traversé par un premier canal axial (19), et un élément postérieur (1b), ou connecteur, traversé par un second canal axial (21), aligné avec le premier canal axial (19), et les moyens d'obturation, et d'aspiration étant regroupés sur l'élément postérieur (1b).

## Claims

1. Surgical knife comprising ultra-sound generator means (3) adapted to subject a sonotrode (9), disposed at its front end, to an ultrasonic vibratory movement, the surgical knife being pierced with at least one axial suction channel (19, 21) connecting its front part to its rear part, and comprising means for guiding a laser radiation up to the front end of the sonotrode, characterized in that it comprises means for directing said laser radiation across the suction channel (19, 21) and for focussing it, at a focussing point (A) located in front of the front end of the sonotrode (9).

2. Surgical knife according to Claim 1, characterized in that it comprises a secondary suction conduit (40) opening out in the axial suction channel (21), near said focussing means (31) adapted to direct the laser radiation to a focussing point (A), creating a suction current (F), and means adapted to control obturation of the axial suction channel, disposed upstream of said suction conduit (40) and downstream of said focussing means (31).

3. Surgical knife according to one of the preceding Claims, characterized in that it comprises means for distributing a gaseous flux in the axial suction channel (21), which open out therein, upstream of the suction conduit (40), and which create a gaseous flux (F') antagonistic to the suction current (F).

4. Surgical knife according to Claim 3, characterized in that the means for distributing the flux (F') antagonistic to the suction flux (F) are constituted by a conduit (42) connecting the axial channel (21) to the outside, so as to admit said antagonistic flux by the Venturi effect.

5. Surgical knife according to one of the preceding Claims, characterized in that the surgical knife is essentially constituted by two separate, connectable elements, namely a front element (1a) or hand piece traversed by a first axial channel (19), and a rear element (1b), or connector, traversed by a second axial channel (21), aligned with the first axial channel (19), and the obturation and suction means being grouped on the rear element (1b).

## Patentansprüche

1. Skalpell, das eine Ultraschallerzeugungseinrichtung (3), die geeignet ist, eine Sonotrode (9), die an ihrem vorderen Ende angeordnet ist, einer vibrierenden Ultraschallbewegung zu unterwerfen, wobei das Skalpell von mindestens einem axialen Saugkanal (19, 21) durchbohrt ist, der seinen vorderen Abschnitt mit seinem hinteren Abschnitt verbindet, und eine Einrichtung aufweist, die geeignet ist, eine Laserstrahlung bis zum vorderen Ende der Sonotrode zu leiten, dadurch gekennzeichnet, daß es eine Einrichtung aufweist, um die Laserstrahlung durch den Saugkanal (19, 21) hindurch zu lenken und um sie in einem Fokussierpunkt (A) zu bündeln, der vor dem vorderen Ende der Sonotrode (9) gelegen ist.

2. Skalpell nach Anspruch 1, dadurch gekennzeichnet, daß es eine zweite Saugleitung (40), die in den axialen Saugkanal (21) in der Nähe der Fokussiereinrichtung (31) mündet, die geeignet ist, die Laserstrahlung in einen Fokussierpunkt (A) zu lenken, wobei ein Saugstrom (F) entsteht, und eine Einrichtung aufweist, die geeignet ist, die Verschließung des axialen Saugkanals zu steuern und stromauf der Saugleitung (40) und stromab der Fokussiereinrichtung (31) angeordnet ist.

3. Skalpell nach einem der vorhergehenden Ansprüche, dadurch gekennzeichnet, daß es eine Einrichtung zur Verteilung eines Gasstroms in dem axialen Saugkanal (21) aufweist, die in diesen stromauf der Saugleitung (40) mündet und einen Gasstrom (F') erzeugt, der dem Saugstrom (F) entgegenwirkt.

4. Skalpell nach Anspruch 3, dadurch gekennzeichnet, daß die Einrichtung zur Verteilung des dem Saugstrom (F) entgegenwirkenden Stroms (F') aus einer Leitung (42) gebildet ist, die den axialen Kanal (21) mit der Außenseite derart verbindet, daß der entgegenwirkende Strom durch den Venturieffekt zugeführt wird.

5. Skalpell nach einem der vorangehenden Ansprüche, dadurch gekennzeichnet, daß das Skalpell im wesentlichen aus zwei getrennten, steckbaren Gliedern, nämlich einem vorderen Glied (1a) oder Handstück, das durch einen ersten axialen Kanal (19) durchquert ist, und einem hinteren Glied (1b) oder Verbindungsstück, das durch einen zweiten axialen Kanal (21) durchquert ist, der mit dem ersten axialen Kanal (19) ausgerichtet ist, und einer Verschluß- und Saugeinrichtung gebildet ist, die auf dem hinteren Glied (1b) gruppiert sind.
